Europäisches Patentamt

European Patent Office

Office européen des brevets

(11) Publication number: **0 230 456 B1**

## EUROPEAN PATENT SPECIFICATION

(45) Date of publication of patent specification: **15.01.92**    (51) Int. Cl.5: **A61K 39/09, C12N 1/20**

(21) Application number: **86904663.1**

(22) Date of filing: **14.07.86**

(86) International application number:
**PCT/US86/01460**

(87) International publication number:
**WO 87/00436 (29.01.87 87/03)**

The file contains technical information submitted
after the application was filed and not included in
this specification

(54) **THE PROTECTION OF EOUINES AGAINST STREPTOCOCCUS EOUI.**

(30) Priority: **12.07.85 US 754613**

(43) Date of publication of application:
**05.08.87 Bulletin 87/32**

(45) Publication of the grant of the patent:
**15.01.92 Bulletin 92/03**

(84) Designated Contracting States:
**AT BE CH DE FR GB IT LI LU NL SE**

(56) References cited:

**Commonwealth Agricultural Bureau, Veterinary Bulletin n OV056-03525, ref. n 86163353. J.F. Timoney et al "The protective response of the horse to an avirulent strain of Streptococcus equi" & Proc. IXth lancefield int. symp. on streptococci and streptococal diseases, September 1984, Japan**

**See also references of WO8700436**

(73) Proprietor: **CORNELL RESEARCH FOUNDATION, INC.**
**East Hill Plaza**
**Ithaca, N.Y. 14850(US)**

(72) Inventor: **TIMONEY, John, F.**
**120 Ludlowville Road**
**Lansing, NY 14882(US)**

(74) Representative: **West, Vivien et al**
**SmithKline Beecham, Corporate Patents,**
**Great Burgh, Yew Tree Bottom Road**
**Epsom, Surrey KT18 5XO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

BACKGROUND OF THE INVENTION

This invention relates to the immunization of equines against Streptococci equi. S. equi causes strangles, an acute upper respiratory tract disease of horses, characterized by fever, nasal discharge and abscess formation in the retropharyngeal and mandibular lymph nodes. Horses that have been so infected in the field or experimentally infected with strangles and which do recover from strangles become highly resistant to reinfection. Moreover, only one antigenic type of S. equi has been observed in the field.

The above notwithstanding, vaccines prepared from bacterins of S. equi, or fractional extracts of the same, such as M protein-rich extracts, have been relatively ineffective to provide protection against S. equi in the field. This is true even though as far back as 1943 an article entitled "Studies with Equine Streptococcus" published in the Australian Veterinary Journal at 19:62 by p. O. Bazeley, presented a broad-range study of the problem coupled with test results which Dr. Bazeley and other characterized as very hopeful. However, many years have passed without an adequate or effective method or means for protection of equines against strangles. One of the problems with earlier experimentation in the field was that scientists and researchers equated protection of the horse against S. equi with stimulation of bactericidal antibodies in the blood serum of the horse. In fact, vaccine failure was not due to failure of vaccines to stimulate bactericidal antibody in the serum, which it was shown did not equate with protection against field or experimental exposure to S. equi. In fact, it was discovered that ponies recently recovered from experimentally induced strangles were highly resistant to reinfection before serum bactericidal activity could be detected. Moreover, it was determined that the nasopharyngeal mucus of resistant ponies contained major IgG and IgA antibody activity against only one acid extract protein of about 41,000 molecular weight (mw), whereas serum antibodies had a number of major specificities. These findings suggested that successful vaccination requires stimulation of the nasopharyngeal immune response.

The following publications have been made by the inventor herein relating to this development:
1) Abstract No. 172 appearing Abstracts IXth, Lancefield International Symposium on Streptococci and Streptococcal Diseases, Fuji, Japan, September 10, 1984;
2) Infection and Immunity, March 1985, Vol. 47, No. 3, pages 623-628;
3) Infection and Immunity, April 1985, Vol. 48, No. 1, pages 29-34.

BRIEF DESCRIPTION OF DRAWINGS

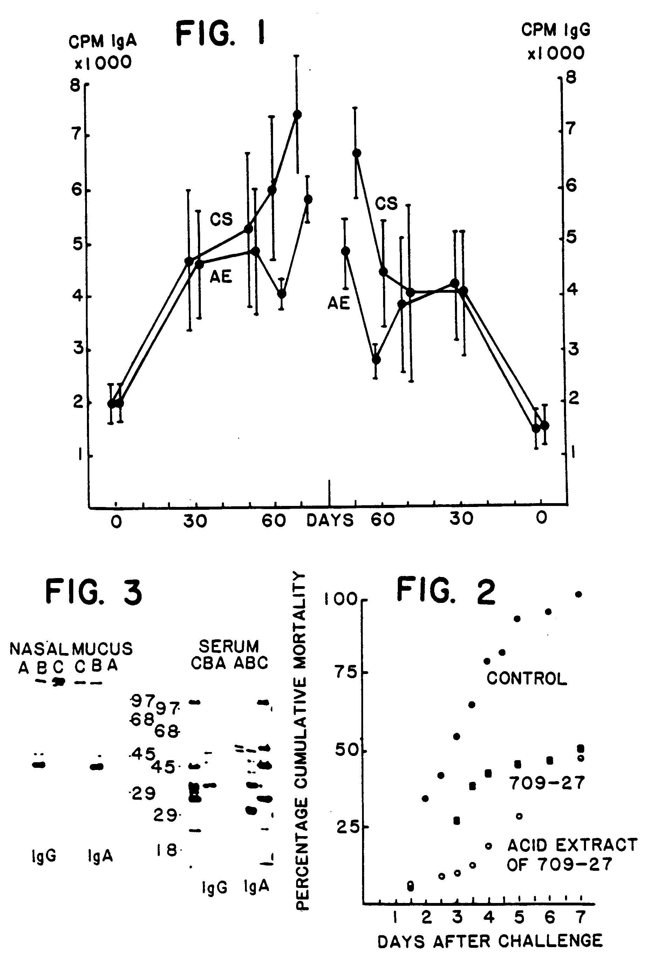

Figure 1 is a graph with separate coordinates for the IgA and IgG antibody titers in nasal washes of 14 ponies against days which have passed following immunization with S. equi 709-27. The antigens in the radioimmunoassay were acid extract and culture supernatant (the native form) protein of S. equi.

Figure 2 is a graph of cumulative mortality against days after challenge for groups of 40 mice vaccinated with live S. equi, 709-27, or an acid extract of S. equi 709-27 and a group of control non-immunized mice. All mice were challenged with 5 x 10-7-CFU virulent S. equi CF32. The information of Fig. 2 is important because it shows that S. equi 709-27 carries the intact M protein, similar to that of the parent S. equi CF32.

Figure 3 is an immunoblot showing proteins (SDS PAGE), S. equi and S. zooepidemicus recognized by IgG and IgA in nasopharyngeal mucus and in serum of a pony following intranasal vaccination with S. equi 709-27. The blots were washed in monospecific antisera against equine IgA or IgG following treatment with nasopharyngeal mucus or serum.
Tracks:
A - Acid extract of S. zooepidemicus
B - Acid extract of S. Equi (CF32)
C - Culture supernatant protein of S. equi (CF32)
Antigens of S. zooepidemicus were included because most horses carry this organism in the nasopharynx and therefore are stimulated to make antibodies to its proteins, some of which are common to S. equi.

The measurement technique described in the Figures are similar to those discussed in the following publications:
a) Infection and Immunity Vol. 47, No. 3 pages 623-628 (March 1985);
b) Infection and Immunity Vol. 48, No. 1 pages 29-34 (April 1985).

DESCRIPTION OF THE INVENTION

The present invention teaches how to stimulate the nasopharyngeal immune response, for example using a bacterial clone derived from a highly virulent strain of S. equi known as S. equi CF32 which is on deposit at the American Type Culture Collection (A.T.C.C. No. 53185) Rockville, Maryland, deposited (11 July 1985). CF32 produces large (1-3 mm) transparent, mucoid colonies that tend to flow together and are surrounded by a wide zone (5-10 mm) of beta hemolysis. Also on deposit with the American Type Culture Collection is a derivative of S. equi CF32 which has been rendered avirulent according to the teachings of the present invention. The avirulent derivative S. equi bacterium is known as Cornell S. equi 709-27 and is available through the A.T.C.C. under A.T.C.C. No. 53186 (deposited 11 July 1985). Cornell 709-27 produces a small (0.5 mm in diameter white, convex smooth surfaced colony surrounded by a narrow (1 mm) zone of beta hemolysis.

This invention relates to an equine vaccine against S. equi caused strangles in an equine, which vaccine stimulates a nasopharyngeal immuno response in a strangles susceptible equine and which vaccine comprises an avirulent strain of S. equi formed by mutating a virulent strangles causing S. equi strain to render it avirulent while retaining thereon protein which provides an acid extract M protein fragment with a molecular weight of about 41,000 which stimulates an immunological response to IgG and IgA antibody similar to that in the nasopharyngeal mucus of an equine recovered from S. equi caused strangles.

The vaccine of the invention is not strain specific. Only one antigenic type of S. equi has been observed in the field. Thus, the method of the invention can be applied to any virulent strangles causing S. equi strain.

The virulent S. equi strain can be rendered avirulent in any manner so long as the resultant avirulent S. equi strain retains the M protein fragment having a molecular weight of about 41,000 which stimulates an immunological response similar to that in the nasopharyngeal mucus of an equine recovered from S. equi caused strangles. The presently preferred method is deliberatedly induced mutagenesis for example by the use of chemicals or radiation. Particularly useful is chemical mutagenesis for example through the use of nitrosoguanidine. (See Chapter 13, Gene Mutation, Manual of Methods of General Bacteriology, American Society for Microbiology, Washington, D.C. 1981).

For the purposes of characterizing the vaccine of the invention through radio-immunoassay or immunoblotting assay the acid extract protein is isolated following techniques described in a publication by R.C. Lancefield entitled "The Antigenic Complex of Streptococcus Hemolyticus I Demonstration of a Type Specific Substance in Extracts of Streptococcus Hemolyticus" J. Exp. Med. 47:91.

For the purpose of further characterizing the vaccine of the invention protein molecular weight is determined by SDS - PAGE Electropheresis and the use of molecular weight standards.

In accordance with the teachings of the present invention a successful vaccine against S. equi requires stimulation of the nasopharyngeal immune response in a susceptible equine by intranasal or oral inoculation. Antibody activity in the nasopharyngeal mucus correlates with protection against strangles, and antibody activity in the blood serum is of less significance.

M-protein-rich extracts were relatively ineffective because they did not stimulate a nasopharyngeal immune response of the susceptible equine, although they were effective in producing an immune response in the blood serum of the animal. In order to stimulate the required response the present invention teaches a method of making avirulent S. equi bacteria which may be used as a vaccine and applied either intranasally or orally and produces major IgG and IgA antibody responses in the nasopharyngeal mucus of the susceptible equine. The avirulent strain of S. equi (Cornell 709-27) is such a bacteria. Hereinafter that especially made bacteria is called S. equi (Cornell 709-27).

## Method of producing an Effective Avirulent Vaccine Strain of S. equi

The strain of S. equi (Cornell 709-27) avirulent for mice and ponies was obtained in the following manner: the starting bacteria, S. equi CF32 was subjected to nitrosoguanidine mutagenesis following the teachings set out in an article by Carlton, B.C. and Brown, B.J. (1981) in Manual of Methods for General Bacteriology. (Eds. P. Gerhardt, et al.) American Society for Microbiology, Washington, D.C., p. 226. Modification of the procedure setforth in the first column of page 226 was undertaken. Specifically, Todd Hewitt broth was used throughout the procedures as a growth medium. Nonencapsulated colonies were screened for loss of virulence by intraperitoneal inoculation of mice (ICR). The strains which did not kill mice were considered positive strains. The positive mouse strains were then used to vaccinate mice by the intraperitoneal route to determine their protective quality. Those strains which were protective of mice were inoculated intranasally into horses. Finally, as described herein, a derived strain of S. equi 709-27 was found to be avirulent in a dose of 3 x 10⁹ CFU, and efficacious as a vaccine against S. equi in susceptible equine when it was intranasally or orally inoculated in the equine. Moreover, the positive strain which also protected equines tested for the presence of the 41k fragment of the M protein by immunoblotting. The identifying number for that strain is S. equi 709-27.

An acid extract of strain S. equi (Cornell 709-27) was shown by immunoblotting to carry the same immunologically reactive proteins as the parent S. equi strain (CF32). The immunoblotting procedure used was similar to that used in a scientific article entitled "Infection and Immunity" Vol. 48, No. 1 pages 29-34 (April 1985).

Equine Immunization and Challenge

The S. equi (Cornell 709-27) was then tested for efficacy as a vaccine against experimental S. equi infection in equine. The following table depicts that testing.

Table 1. Resistance of Ponies to Intranasal Challenge with Streptococcus equi Following Intranasal Immunization with the Avirulent Strain of S. equi 709-27.

| Treatment (vaccinate with S. equi 709-27) | Challenge (CFU Virulent S. equi CF32) | No. Ponies | No. Resistant |
|---|---|---|---|
| $3 \times 10^9$ | $5 \times 10^8$ | 14 | 14 |
| Day 0 and Day 30 | Day 59 | | |
| Contact Controls | " | 2 | 2 |
| Isolation Controls | " | 6 | 0* |

*All controls developed acute strangles within 4 days of challenge.

Fourteen yearling ponies raised in isolation and never exposed to S. equi were given an atomized suspension (intranasally) of an 18 hour culture ($3\ 10^9$ CFU) in Todd Hewitt broth of 709-27. A repeat inoculation was given 29 days later. Ponies were challenged intranasally 30 days later with $5 \times 10^8$ CFU of an overnight culture of S. equi CF32. Cultures were administered by means of a nasal atomizer (Model #281, Devilbiss Co., Somerset, PA).

Six non-vaccinated ponies housed separately from the vaccinated group and 2 contact control ponies were also challenged with the same CF32 inoculum. All of the immunized ponies and the 2 contact control ponies were resistant to S. equi when challenged, but all of the isolation controls developed acute strangles within 4 days of challenge.

In addition about 800 horses on farms with endemic S. equi infection problems were experimentally intranasally or orally vaccinated with S. equi 709-27 to date and only two horses have developed strangles. The expected occurrence of strangles on those farms based on the experience of the three previous years, is such that one would have predicted the occurrence of strangles in the range of 40% of the horses.

When using the teachings of the present invention to vaccinate horses against S. equi the results of oral inoculation appeared to be comparable with intranasal inoculation with the same dose. The vaccine dose (number of organisms) used in the vaccination described herein was 100 times greater than the number of organisms of a wild virulent strain of S. equi (CF32), which would be expected to cause disease in a normal equine. However, a commercial S. equi vaccine program would utilize dosage levels which were determined by consultation between the manufacturer and the appropriate governmental authorities.

Freezing or freeze drying does not adversely affect the vaccine. These procedures can therefore be used in mass production and distribution of the vaccine.

The vaccine has been entirely without side effects in adult animals, but a low (~5°) incidence of submandibular abscesses has been observed on one occasion in 3-month foals. This adverse reaction occurred when a very heavy dose of vaccine was administered in an effort to obtain consistent seroconversion in the blood serum of the inoculated equine. As stated elsewhere, it is nasopharyngeal mucus of the susceptible equine that contains antibodies involved in immunological protection.

Antibody Assays - Figure 1

IgA and IgG antibodies to the proteins of S. equi (CF32) were assayed in sera and nasal washes collected before, during, and after vaccination and challenge. Assays were performed by solid phase radioimmunoassay as described in an article entitled "Immunochemical Quantitation of Antigens by Single Radial Immunodiffusion" by G. Mancini, A.O. Carbonara and J.H. Heremans in Immunochemistry 2: pages 235-254. Wells were coated with acid extract (AE) or culture supernatant (CS) protein of S. equi.

IgA and IgG antibody responses to acid extract and culture supernatant proteins of S. equi were observed in nasal washes from all vaccinated animals (Figure 1). Serum antibody responses were also observed, but they were inconsistent. Contact control ponies showed nasal and serum antibody conversion at the same time - an indication that transmission of the vaccine strain had occurred in the group. Principal and contact control ponies were resistant to challenge with virulent S. equi whereas non-vaccinated ponies developed typical strangles within 4 days of challenge (Table 1).

Mouse Immunization and Challenge - Figure 2

The mouse has historically been the model for the immunology of S. equi infection. Accordingly, as a parallel test of efficacy, adult ICR mice were immunized subcutaneously with hydroxyapatite purified protein of an acid extract of S. equi 709-27. Reference is made to an article by Vosti, K.L. Journal of Med. Microbiol. 11:453 (1978). Protein was absorbed to aluminum hydroxide and administered in two subcutaneous doses of 50 $\mu$g 21 days apart. All mice, including a group of non-vaccinated controls, were later challenged with virulent S. equi (5 x 10$^7$ CFU) given intraperitoneally. Mouse mortality was recorded for 7 days following challenge. The difference in mortality between the control and vaccinated groups was highly significant using the Chi square analysis used in statistics. The mice immunized either with an acid extract or live cells of S. equi 709-27 showed a significant protective response (probability $\leq$ .01) as compared with non-vaccinated controls (Figure 2). This result suggested that S. equi 709-27 retained the protective M antigen of S. equi.

Notwithstanding the fact that it is not virulent, an acid extract of S. equi 709-27 was shown by immunoblotting to carry the same immunologically reactive proteins as the parent S. equi strain.

Fig. 3 Immunoblotting Showing Proteins Recognized by Mucosal and Serum Antibodies

The immunologically reactive proteins in an acid extract and culture supernatant of S. equi and an acid extract of S. zooepidemicus were distinguished on nitrocellulose blots of SDS - PAGE gels. Blots were treated with sera or nasopharyngeal mucus collected when ponies were killed 7 days after challenge. A scientific article entitled "Infection and Immunity" Vol. 47, No. 3 pages 623-628 (March 1985) describes the technique used.

Immunoblotting revealed that IgA and IgG antibodies in nasopharyngeal mucus of vaccinated animals were directed mainly against a 41k M protein fragment, whereas serum antibodies had a much broader spectrum of activity, a finding noted previously in ponies following recovery from experimentally induced strangles. Since an antibody response to the 41,000 mw M protein fragment is a constant feature of the nasopharyngeal immune response of resistant horses, it is an important protective antigen.

The antibody response is also specific to S. equi because similarly reactive proteins of S. zooepidemicus could not be detected on the immunoblot (Track A). Other studies have indicated that antibodies in strongly bactericidal sera react strongly with M protein fragments of about 29,000 and 37,000 molecular weight. A hypothesis to explain the different molecular weights of the M protein fragments of S. equi recognized by serum and nasopharyngeal antibody is that the portion or region of the M protein molecule of S. equi important in the nasopharyngeal response, differs from that involved in the stimulation

5

of bactericidal antibody in serum.

In summary, the present invention teaches a new and improved bacterial vaccine to protect susceptible equine against S. equi which causes strangles. The vaccine stimulates a nasopharyngeal immune response in a susceptible equine through the presence of antibody in the nasopharyngeal mucus. The vaccine is a S. equi strain which contains an M-protein fragment of 41,000 mw and is adapted for administration to equine either intranasally or orally as a vaccine. The teachings of the present invention include: a new strain of S. equi (709-27), a method of making and isolating useful vaccine strain of S. equi bacteria, and which stimulates an antibody response in the nasopharyngeal mucosa of the susceptible equine.

Accordingly, it is to be understood that the embodiments of the invention herein described are merely illustrative of the application of the principles of the invention. Reference herein to details of the illustrated embodiments are not intended to limit the scope of the claims which themselves recite those features regarded as essential to the invention.

**Claims**

1. A vaccine for protecting equines against S. equi caused strangles which comprises an avirulent strain of S. equi which stimulates an antibody response in the nasopharyngeal mucosa of the susceptible equine, characterised in that the strain of avirulent S. equi is S. equi 709-27 (ATCC Accession Number 53186), is nonencapsulated, and includes an M-protein fragment with a molecular weight of about 41,000.

2. A vaccine for protecting equines against S. equi caused strangles which comprises an avirulent strain of S. equi which stimulates an antibody response in the nasopharyngeal mucosa of the susceptible equine, characterised in that the strain of avirulent S. equi is S. equi 709-27 (ATCC Accession Number 53186), includes an M-protein fragment with a molecular weight of about 41,000, and which can be inoculated intranasally or orally.

3. A vaccine for protecting equines against S. equi which comprises an avirulent strain of S. equi known as S. equi 709-27 (ATCC Accession Number 53186) which can be inoculated intranasally or orally in the susceptible equine.

4. A method of making a strain of S. equi which is avirulent for equines comprising of the following steps:
   1. subjecting a virulent strain of S. equi -CF32 (ATCC Accession Number 53185) to mutagenesis;
   2. selecting a resulting bacterium which provides an M-protein fragment having a molecular weight of 41,000, which bacterium produces an S. equi antibody response in the nasopharyngeal mucus of an S. equi susceptible equine.

5. S. equi strain 709-27 (ATCC Accession Number 53186) for use in therapy.

6. S. equi strain 709-27 (ATCC Accession Number 53186) for use in the manufacture of a medicament for use in the treatment of strangles in equines.

7. S. equi strain 709-27 (ATCC Accession Number 543186).

**Revendications**

1. Vaccin pour protéger les chevaux contre la gourme provoquée par le S. equi, qui comprend une souche avirulente de S. equi qui stimule la réponse d'un anticorps dans la muqueuse nasopharyngienne du cheval réceptif, caractérisé en ce que la souche de S. equi avirulent est le S. equi 709-27 (numéro d'accession ATCC 53186), il est non-encapsulé et englobe un fragment de protéine M d'un poids molécu-laire d'environ 41.000.

2. Vaccin pour protéger les chevaux contre la gourme provoquée par le S. equi, qui comprend une souche avirulente de S. equi qui stimule la réponse d'un anticorps dans la muqueuse nasopharyngienne du cheval réceptif, caractérisé en ce que la souche de S. equi avirulent est le S. equi 709-27 (numéro d'accession ATCC 53186), englobe un fragment de protéine M d'un poids moléculaire d'environ 41.000 et peut être inoculé par voie intranasale ou orale.

6

**3.** Vaccin pour protéger les chevaux contre le S. equi, qui comprend une souche avirulente de S. equi, connue en tant que S. equi 709-27 (numéro d'accession ATCC 53186), qui peut être inoculé par voie intranasale ou orale chez le cheval réceptif.

**4.** Procédé de préparation d une souche de S. equi qui est avirulente pour les chevaux, comprenant les opérations suivantes :

1. soumettre une souche virulente de S. equi-CF 32 (numéro d'accession ATCC 53185) à une mutagénèse ;

2. choisir une bactérie résultante qui donne un fragment de protéine M d'un poids moléculaire de 41.000, laquelle bactérie produit une réponse de l'anticorps du S. equi dans la muqueuse nasopharyngienne d'un cheval réceptif au S. equi.

**5.** Souche de S.equi 709-27 (numéro d'accession ATCC 53186), pour une utilisation thérapeutique.

**6.** Souche de S.equi 709-27 (numéro d'accession ATCC 53186), pour une utilisation dans la fabrication d'un médicament utilisé dans le traitement de la gourme chez les chevaux.

**7.** Souche de S.equi 709-27 (numéro d'accession ATCC 53186).

**Patentansprüche**

**1.** Impfstoff zum Schutz von Pferden gegen durch S. equi verursachte Druse, enthaltend einen avirulenten S. equi-Stamm, der eine Antikörperreaktion in der Nasenrachenschleimhaut des empfänglichen Pferdes stimuliert, **dadurch gekennzeichnet,** daß der avirulente S. equi-Stamm S. equi 709-29 (ATCC-Hinterlegungs-Nummer 53186) ist, nicht verkapselt ist und ein Fragment des M-Proteins mit einem Molekulargewicht von etwa 41 000 enthält.

**2.** Impfstoff zum Schutz von Pferden gegen durch S. equi verursachte Druse enthaltend einen avirulenten S. equi-Stamm, der eine Antikörperreaktion in der Nasenrachenschleimhaut des empfänglichen Pferdes stimuliert, **dadurch gekennzeichnet,** daß der avirulente S. equi-Stamm S. equi 709-27 (ATCC-Hinterlegungs-Nr. 53186) ist, ein Fragment des M-Proteins mit einem Molekulargewicht von etwa 41 000 enthält und intranasal oder oral verimpft werden kann.

**3.** Impfstoff zum Schutz von Pferden gegen S. equi enthaltend einen avirulenten Stamm von S. equi, der S. equi 709-27 (ATCC-Hinterlegungs-Nummer 53186) ist und dem empfänglichen Pferd intranasal oder oral verimpft werden kann.

**4.** Verfahren zur Herstellung eines für Pferde avirulenten S. equi-Stammes, wobei man die folgenden Schritte durchführt:

1. ein virulenter Stamm von S. equi-CF32 (ATCC-Hinterlegungs-Nummer 53185) wird einer Mutagenese unterworfen;

2. ein so erhaltenes Bakterium, das ein Fragment des M-Proteins mit einem Molekulargewicht von 41 000 aufweist, wird ausgewählt, wobei das Bakterium eine Antikörperreaktion gegen S. equi in der Nasenrachenschleimhaut eines für S. equi empfänglichen Pferdes erzeugt.

**5.** S. equi-Stamm 709-27 (ATCC-Hinterlegungs-Nummer 53186) zur Verwendung in der Therapie.

**6.** S. equi-Stamm 709-27 (ATCC-Hinterlegungs-Nummer 53186) zur Verwendung in der Herstellung eines Arzneimittels zur Verwendung in der Behandlung von Druse bei Pferden.

**7.** S. equi-Stamm 709-27 (ATCC-Hinterlegungs-Nummer 53186).

# FIG. 1

CPM IgA
x1000

CPM IgG
x1000

CS

AE

AE

CS

DAYS

# FIG. 3

NASAL MUCUS
A B C   C B A

SERUM
C B A   A B C

97 97
68 68

45
45

29
29

18

IgG   IgA

IgG   IgA

# FIG. 2

PERCENTAGE CUMULATIVE MORTALITY

100

75

CONTROL

50

709-27

25

ACID EXTRACT
OF 709-27

DAYS AFTER CHALLENGE